# EUROPÄISCHE PATENTSCHRIFT

(11) **EP 1 741 460 B1**
(45) Veröffentlichungstag und Bekanntmachung des Hinweises auf die Patenterteilung: **19.09.2007**
(21) Anmeldenummer: 05014700.8
(22) Anmeldetag: 06.07.2005
(51) Int. Cl.: A61M 15/00, A61M 39/22

(54) **Regelbares Ventil und Inhalationsvorrichtung**
Adjustable valve and inhaler device
Soupape réglable et dispositif d'inhalation

(43) Veröffentlichungstag der Anmeldung: 10.01.2007
(73) Patentinhaber: Activaero GmbH, 35285 Gemünden (DE)
(72) Erfinder: Roeder, Sascha, 85053 Ingolstadt (DE); Kolb, Tobias, 80687 München (DE)
(74) Vertreter: Vossius & Partner

(56) Entgegenhaltungen:
- EP-A- 1 163 921
- WO-A-95/05208
- WO-A-03/053502
- WO-A-03/090842
- US-A- 4 319 566
- US-A- 5 937 855

## Beschreibung

Die Erfindung betrifft ein regelbares Ventil und eine Vorrichtung zum Inhalieren von dosierten Arzneistoffen in Aerosolform in die Lunge. Geeignete Arzneistoffe umfassen Analgetika, Antianginamittel, Antiallergica, Antihistamine und entzündungshemmende Mittel, Expectorantia, Antitussiva, Bronchodilatoren, Diuretika, Anticholinergica, Corticoide, Xanthine, Antitumormittel, therapeutisch wirksame Proteine oder Peptide wie Insulin oder Interferon, Antioxidanzien, anti-inflamatorische Substanzen, Wirkstoffe bzw. Medikamente sowie Kombinationen davon.

Bevorzugt ist die Verabreichung von Arzneistoffen zur Behandlung von respiratorischen Erkrankungen wie Asthma, sowie Mittel zur Prophylaxe und Behandlung der Schleimhäute des Tracheo-Bronchialtraktes. Hier ist die Verabreichung von Kortikoiden ist möglich.

Weiter bevorzugtes Anwendungsgebiet ist die variable Flussbegrenzung bei Lungendiagnosegeräten. Dies ist denkbar bei allen Messverfahren, welche zum Beispiel Aerosolteilchen für die Diagnose nützen.

Die EP-A-1163921 offenbart eine Vorrichtung zur Flussbegrenzung bei niedrigen Differenzdrücken, insbesondere zur Begrenzung des Inhalationsvolumenstroms bei einer Inhalation von therapeutischen Aerosolen. Die Vorrichtung besteht aus einem Gehäuse mit einer Einsaugöffnung, einer Aussaugöffnung und einem zwischen diesem angeordneten Strömungskanal mit einem flachen, länglichen Querschnitt mit flexiblen großflächigen Wänden. Der Querschnitt des Strömungskanals ist abhängig von dem Differenzdruck zwischen Aussaugöffnung und Einsaugöffnung sowie der Materialflexibilität des Wandmaterials zu einer Größe für einen vorgegebenen maximalen Inhalationsvolumenstrom verringerbar.

Die Verabreichung von Arzneistoffen in Aerosolform durch Inhalation in die Lunge ist im wesentlichen durch vier Faktoren beeinflusst: (i) die Partikelgröße und die Partikeleigenschaften des Aerosols; (ii) das Atemzugvolumen des Patienten; (iii) der Atemfluss des Patienten; und (iv) die Morphometrie und Atemwege des Patienten. Während bisherige Systeme zwar Aerosole in geeigneten Teilchengrößenbereichen produzieren, werden bei bekannten Systemen die Parameter "Atemzugvolumen" und "Atemfluss" (Atemgeschwindigkeit) nur ungenügend oder gar nicht berücksichtigt. Dies führt zu einer unkontrollierten Inhalation des Aerosols, was wiederum dazu führt, dass eine unzureichende Menge an Aerosolteilchen die Lunge erreicht bzw. innerhalb der Lunge die zu therapierenden Bereiche (beispielsweise Alveolarbereich) nicht erreicht.

In der EP-A-0 965 355 wird eine Vorrichtung zum gesteuerten inhalatorischen Einbringen von Dosiermedikamenten in die Lunge vorgeschlagen. Dieser gesteuerte Inhalator weist einen geschlossenen Behälter auf, der mit einem vorbestimmbaren Aerosolvolumen befüllbar ist und aus dem das Aerosol mittels einer Steuereinrichtung für den Inhalationsfluss entnehmbar ist. Bei diesem bekannten Inhalator ist diese Steuereinrichtung entweder ein Verstellventil oder eine kritische Düse. Durch die Verwendung eines verstellbaren Ventils oder einer kritischen Düse kann eine Atemflusslimitierung erreicht werden.

In der EP-B-0 050 654 wird ein Atemgerät zur medikamentösen Behandlung der Lunge vorgeschlagen. Dieses Atemgerät weist eine aufblasbare Hülle auf, aus der mittels eines Mundstückes Aerosol inhaliert werden kann. Dieses Aerosol wird vor der Inhalation aus einer Kartusche über einen Zerstäuber in die aufblasbare Hülle eingebracht. Zur Begrenzung der Durchflussmenge der Luft durch das Mundstück bei der Inhalation weist das Mundstück eine Verengung auf. Diese Verengung limitiert den Atemfluss bei der Inhalation.

Die beiden genannten Inhalationsvorrichtungen zeichnen sich dadurch aus, dass eine FlussLimitierung erfolgt, d. h. während der Inspirationsphase steigt der Atemfluss lediglich langsam an und der Anstieg des Atemflusses nimmt stetig ab, was bei graphischer Darstellung des Atemflusses gegenüber der Zeit zu einer ständigen Abflachung der Kurve führt. Diese Flusslimitierung führt dazu, dass je nach Saugvermögen des Patienten der Atemfluss unterschiedlich bis auf einen maximalen Flusswert ansteigt. Der Fluss wird so nahezu konstant gehalten. Dies bedeutet, dass bei den bekannten Inhalatoren die vorgesehene Flusslimitierung zu einer konstanteren Aerosoldeposition in der Lunge führen kann.

Die EP-A-1 036 569 offenbart ein Verfahren und eine Vorrichtung zur Bereitstellung einer konstanten Medikamenten-Dosis für eine inhalatorische Applikation mit niedrigem Inhalationsfluss. Diese Vorrichtung besteht aus einem volumenreduzierbaren, geschlossenen Behälter, einem mit dem Behälter verbundenen Mundstück, an dem zur Aerosol-Bereitstellung ein Pulver-Aerosol-Inhalator anschließbar ist, einem den Behälter geschlossen umgebendes, volumenreduzierbares Gehäuse, aus dem das Mundstück abgedichtet herausgeführt ist, und aus einer Einrichtung zur Steuerung des Ein- und Auslassens von Luft in den bzw. aus dem Bereich zwischen dem Behälter und dem Gehäuse. Das Gehäuse ist aus einem volumenreduzierten Zustand in einen vorgesehenen, expandierten Bereitstellungszustand zur Befüllung des Behälters mit dem vorgesehenen Aerosolvolumen bringbar.

Des weiteren offenbart die EP-A-1 038 544 eine Vorrichtung zur Flussbegrenzung bei niedrigen Differenzdrücken, insbesondere für das Begrenzen des Inhalationsvolumenstroms bei der Inhalation von therapeutischen Aerosolen. Diese Vorrichtung besteht aus einem Gehäuse mit wenigstens einer Einsaugöffnung, wenigstens einer Aussaugöffnung und einem zwischen diesen angeordneten Strömungsbereich mit wenigstens einer flexiblen Wand, deren Querschnitt abhängig von dem zwischen Ansaugöffnung und Einsaugöffnung herrschenden Unterdruck und der Materialflexibilität des Wandmaterials bis zu einer vorbestimmten Größe für einen vorgegebenen maximalen Inhalationsvolumenstrom verringerbar ist.

Ferner offenbart die US-A-5 655 520 ein flexibles Ventil zur Verabreichung von Medikamenten mit konstanter Strömungsgeschwindigkeit.

Die US-A-5 842 467 offenbart eine Inhalationsvorrichtung zur Abgabe vorbestimmter Dosen von Medikamenten.

Die EP-A-1 136 921 offenbart eine Inhalationsvorrichtung mit einem selbst expandierbaren Behältnis für ein vorbestimmtes Aerosolvolumen, einer Einrichtung zum Einbringen von Aerosol aus einem Aerosolspender in das Behältnis und einer Steuereinrichtung zum Steuern des Inhalationsflusses. Die Steuereinrichtung hält den Inhalationsfluss während der gesamten Inhalation des Aerosols im wesentlichen konstant, wobei die Steuereinrichtung vier zwischen einer zentralen Einlassöffnung und zu der Einlassöffnung radial beabstandeten Auslassöffnungen radial verlaufende Strömungskanäle aufweist. Die vier radial verlaufenden Strömungskanäle sind durch vier sternförmig angeordnete, rechteckige Stege gebildet, die sich von einer im Wesentlichen starren Wand zu einer im Wesentlichen flexiblen Wand erstrecken. Dabei ist ein Steg länger als die anderen.

Der vorliegenden Erfindung liegt die Aufgabe zugrunde, eine verbesserte Steuereinrichtung bzw. ein verbessertes Ventil bereitzustellen, das insbesondere in einer Inhalationsvorrichtung verwendbar ist und unabhängig von Patientencharakteristika den für die Inhalation von Aerosolen notwenigen Atemfluss bereitstellt und gleichzeitig variabel einsetzbar ist. Diese Aufgabe wird durch ein Ventil bzw. eine Inhalationsvorrichtung mit den Merkmalen der Ansprüche gelöst.

Die erfindungsgemäße Inhalationsvorrichtung hält den Inhalationsfluss während der gesamten Inhalation des Aerosols im wesentlichen konstant und weist zur Begrenzung des durch die Steuereinrichtung fließenden Volumenstroms ein regelbares Ventil auf. Dieses Ventil ist vorzugsweise in Form eines variablen Flussbegrenzers, der beispielsweise eine stufenlose Flussregelung oder eine gestufte Flussregelung ermöglicht, realisierbar.

Das erfindungsgemäße regelbare Ventil weist vorzugsweise ein Gehäuse, einen Stellkolben, eine flexible Wand (beispielsweise eine Membran bzw. eine Elastomerscheibe), eine optionale Druckplatte, ein Verschlusselement und eine Stellschraube auf. Das Gehäuse ist vorzugsweise im wesentlichen rohrförmig ausgebildet und weist mehrere, zum Beispiel vier sternförmig angeordnete Stege auf, von denen ein Steg länger ist als die anderen. Diese Stege ragen von der rohrförmigen Wandung des Gehäuses radial nach innen. In Axialrichtung betrachtet, sind die Stege in einem Mittelbereich des Gehäuses angeordnet, so dass das Gehäuse einerseits der Stege zur verstellbaren Aufnahme des Stellkolbens und auf der gegenüberliegenden Seite der Stege zur Aufnahme der Membran bzw. Elastomerscheibe, der Druckplatte und des Verschlusselements ausgebildet ist.

Ferner ist die erfindungsgemäße Inhalationsvorrichtung vorzugsweise so konstruiert, dass die Elastomerscheibe mittels der Druckplatte und des Verschlusselements gegen die Stege des Gehäuses gedrängt wird, so dass zwischen der Membran und dem Stellkolben ein Volumenraum gebildet wird. Hierzu weist der Stellkolben vorzugsweise an einer zur Elastomerscheibe gerichteten Stirnfläche mehrere, beispielsweise vier korrespondierend zu den Stegen am Gehäuse sternförmig ausgebildete Ausnehmungen auf, wobei eine Ausnehmung länger ist als die anderen. Zwischen zwei benachbarten Stegen sind vorzugsweise mehrere, beispielsweise zwei Einlassöffnungen vorgesehen, die beispielsweise auf einer gemeinsamen Umfangslinie liegen und jeweils um 45° voneinander beabstandet sind. In einem den Ausnehmungen abgewandten Innenbereich des Stellkolbens münden die Einlassöffnungen in einen gemeinsamen, zentralen Strömungskanal. Hierzu ist der Stellkolben vorzugsweise zweiteilig ausgebildet, wobei ein erster Teil die Ausnehmungen mit den Einlassöffnungen aufweist und ein zweiter Teil, der vorzugsweise mit dem ersten Teil fest verbunden, zum Beispiel verklebt, ist, beinhaltet den zentralen Strömungskanal, mit dem die Einlassöffnungen in Verbindung stehen.

Der Stellkolben ist ferner vorzugsweise dichtend im Gehäuse aufgenommen. Des weiteren ist die Stellschraube vorzugsweise auf dem Stellkolben in Axialrichtung fixierbar, so dass der Stellkolben mittels der Stellschraube relativ zum Gehäuse in Axialrichtung einstellbar ist. Hierzu weist die Stellschraube vorzugsweise ein Außengewinde auf, das mit einem im Gehäuse vorgesehenen, korrespondierenden Innengewinde in Eingriff bringbar ist. Durch Verstellen der Stellschraube lässt sich der Kolben somit axial verschieben, so dass die am Gehäuse vorgesehenen Stege beliebig weit in die am Stellkolben vorgesehenen Ausnehmungen einführbar sind.

Entsprechend einer weiteren Ausführungsform ist das erfindungsgemäße regelbare Ventil stufenweise einstellbar, wozu vorzugsweise eine Kulissenführung zwischen Stellkolben und Gehäuse vorgesehen ist. Im übrigen sind die Komponenten des regelbaren Ventils mit denen in der ersten Ausführungsform im wesentlichen identisch.

Vorzugsweise ist das regelbare Ventil an eine Inhalationsbegrenzung angeschlossen. Die Inhalationsvolumenbegrenzung ist beispielsweise ein Inhalationsbeutel, ein Schaufelrad oder ein Zuluftkanal, der nach einer vorbestimmten Zeit verschlossen wird. Von dem vorgegebenen Fluss und der gemessenen Inhalationszeit kann auf das verabreichte Volumen geschlossen werden. Sobald dies erreicht ist, wird der Zufluss verschlossen. Dies bedeutet, dass die Volumenmessung indirekt über die Messung der Inhalationszeit erfolgt. Hier ist sowohl eine mechanische, als auch eine elektronische Messung des Volumens denkbar.

Eine mit dem erfindungsgemäßen regelbaren Ventil ausgestattete Inhalationsvorrichtung hält während der gesamten Inhalation des Aerosols den Inhalationsfluss im wesentlichen konstant. Dies bedeutet, dass der Inhalationsfluss unmittelbar bei Beginn der Inspirationsphase auf seinen Maximalwert ansteigt, der für die ausreichende Verabreichung des Aerosols notwendig ist und auf diesem Maximalwert bleibt, solange vom Patienten während der Inhalation ein Mindestdruck erzeugt wird. Dieser Mindestdruck beträgt vorzugsweise höchstens 10 mbar und liegt bevorzugt im Bereich zwischen 5 und 10 mbar. Somit wird bereits bei niedrigen Differenzdrücken eine Flussbegrenzung bereitgestellt.

Die Inhalationsvorrichtung ist vorzugsweise eine Kombination aus einem, insbesondere selbstexpandierenden, Behältnis für ein vorbestimmtes Aerosolvolumen, einer Einrichtung zum Einbringen von Aerosol aus einem Aerosolspender in das Behältnis und einer Einrichtung zum Steuern des Inhalationsflusses, wobei die Steuereinrichtung ein erfindungsgemäßes regelbares Ventil aufweist, um den Inhalationsfluss während der gesamten Inhalation im wesentlichen konstant zu halten.

Der Strömungskanal mündet vorzugsweise in das Innere eines Gehäuses, das das Aerosolbehältnis umgibt. Vor der Inhalation des Aerosols wird dieses beispielsweise aus einer Kartusche in das Innere des Behältnisses eingebracht, vorzugsweise über eine Düse, wie etwa einen Vernebler oder ein Dosieraerosol. Dabei expandiert das Behältnis bis sich im voll expandierten Zustand ein durch das Behältnisvolumen vorbestimmtes Aerosolvolumen im Inneren des Behältnisses befindet. Alternativ dazu ist es auch möglich, das Aerosolvolumen direkt an dem Vernebler bzw. an dem Dosieraerosol anzuordnen.

Sobald ein Patient über ein vorzugsweise vorgesehenes Mundstück das Aerosol aus dem Behältnis ansaugt, zieht sich dieses aufgrund des Soges zusammen. Der daraufhin im Gehäuseinneren entstehende Unterdruck wird über den Strömungskanal ausgeglichen. Der anliegende Unterdruck bewirkt dabei, dass sich die flexible Wand abhängig von der Größe des Unterdrucks zum Inneren des Strömungskanals hin wölbt, und dadurch dessen Querschnitt verringert. Diese Querschnittsverringerung bewirkt eine Begrenzung des Luftstromes durch den Strömungskanal in das Gehäuseinnere zum Druckausgleich, was wiederum den Aerosolfluss heraus aus dem Behältnis begrenzt. Durch die Steuereinrichtung setzt bereits bei Drücken von 10 mbar eine selbsttätige Volumenstromregelung im Strömungskanal und somit selbsttätige Atemflussregelung ein.

Der beim Einatmen des Aerosols entstehende Unterdruck bewirkt unmittelbar eine Querschnittsreduzierung des Strömungskanals aufgrund der flexiblen Wand, und zwar eine Reduzierung unmittelbar auf einen Grenzwert. Dies bewirkt, dass der Atemflussgrenzwert bereits unmittelbar bei Beginn der Inhalation erreicht wird und dieser bei durch das Ansaugen von der Lunge üblicherweise hervorgerufenen Drücken von 80 bis 100 mbar während der gesamten Inhalation im wesentlichen beibehalten wird.

Durch die Einrichtung zum Einbringen von Aerosol aus einem Aerosolspender in das Behältnis wird verhindert, dass ein Arzneistoff wie etwa Kortikoide in Form eines Aerosols direkt aus dem Aerosolspender in den Mundraum ausgegeben und inhaliert wird. Vielmehr wird der Patient angehalten, das Aerosol aus dem Aerosolspender in das Behältnis einzubringen, um dann das durch den Behälter definierte vorbestimmte Aerosolvolumen mittels der erfindungsgemäßen Inhalationsvorrichtung zu inhalieren. Vorzugsweise wird der Aerosolspender wie etwa eine Kartusche über einen Kragen mit einer Düse verbunden und an der Inhalationsvorrichtung gehalten. Über die Düse wird das Aerosol in das Innere des Behältnisses eingebracht.

Die erfindungsgemäße Inhalationsvorrichtung weist zahlreiche Vorteile auf. Die erfindungsgemäße Inhalationsvorrichtung erlaubt eine gleichmäßige und präzise Medikamentendosierung unabhängig vom Koordinationsvermögen des Patienten. Durch unterschiedliche Volumina des Behältnisses ist der gewünschte Depositionsort in der Lunge und auch die gewünschte Aerosolmenge vorwählbar. Bei zumindest teilweise durchsichtiger Ausgestaltung des Gehäuses kann das inhalierte Volumen visuell kontrolliert werden, da der Patient sieht, wie sich das Behältnis zusammenfaltet. Die erfindungsgemäße Inhalationsvorrichtung erlaubt eine einfache Handhabung und zugleich hohe Effektivität. Durch das Einbringen des Wirkstoffes in das Behältnis vor der Inhalation wird die Aerosolausgabe aus dem Spender auf die notwendige Menge beschränkt, was einen übermäßigen Verbrauch verhindert. Die genaue und effiziente Dosierung wiederum führt zu niedrigen Kosten der Behandlung beispielsweise mit Kortikoiden. Ein weiterer Vorteil der Erfindung ist, dass die Verwendung eines Treibmittels, beispielsweise für die Verabreichung von Kortikoiden, nicht zwingend erforderlich ist.

Der Begriff "geeignete Arzneistoffe" wie hier verwendet schließt Wirkstoffe, Medikamente, Verbindungen, Zusammensetzungen oder Mischungen von Stoffen ein, die einen pharmakologischen, oft vorteilhaften Effekt erzielen. Dies schließt Speisen, Nahrungsergänzungsstoffe, Nährstoffe, Medikamente, Impfstoffe, Vitamine und weitere nützliche Wirkstoffe mit ein. Die Ausdrücke, wie sie hier benutzt werden, schließen weiterhin alle physiologisch oder pharmakologisch aktiven Substanzen ein, die einen lokalen oder systemischen Effekt in einem Patienten bewirken. Der aktive Wirkstoff, der in Aerosolform zugeführt werden kann, schließt Antikörper, antivirale Wirkstoffe, Antiepileptika, Analgetika, entzündungshemmende Wirkstoffe und Bronchodilatatoren ein und kann eine organische oder anorganische Verbindung sein, die ohne Beschränkungen auch Medikamente einschließt, die auf das periphäre Nevensystem, adrenerge Rezeptoren, cholinerge Rezeptoren, Skelettmuskulatur, kardiovaskuläres System, glatte Muskulatur, Blutkreislaufsystem, neuronale Verbindungen, endokrines- und Hormonsystem, Immunsystem, reproduktives System, Skelettsystem, Nahrungszufuhr- und exkretorisches System, Histaminkaskade oder zentrales Nervensystem wirken. Geeignete Wirkstoffe können z.B. aus Polysacchariden, Steroiden, Hypnotika und Sedativa, antriebssteigernden Mitteln, Beruhigungsmitteln, krampflösenden und muskelentspannenden Mitteln, Antiparkinson-Substanzen, Analgetica, Entzündungshemmern, antimikrobiellen Wirkstoffen, Antimalariamitteln, Hormonen inklusive empfängnisverhütenden Mitteln, Symphaticomimetica, Polypeptiden und Proteinen, die physiologische Effekte hervorrufen, Diuretica, Fettstoffwechsel regulierenden Substanzen, antiandrogenen Wirkstoffen, gegen Parasiten gerichteten Mitteln, neoplastisch und antineoplastisch wirkenden Stoffen, Antidiabetika, Nahrungs- und Nahrungsergänzungsstoffen, wachstumsfördernden Stoffen, Fetten, stuhlregulierenden Stoffen, Elektrolyten, Impfstoffen und Diagnostika bestehen. Auch Kombinationen von Wirkstoffen (Kombinationspräparate) sind möglich. Ferner können auch systemisch wirkende Mittel (z. B. Insulin) verabreicht werden.

Die Erfindung ist besonders geeignet zur inhalativen Applikation von verschiedenen Wirkstoffen (ist aber nicht darauf beschränkt) wie:

Insulin, Calcitonin, Erythropoietin (EPO), Faktor VIII, Faktor IX, Cyclosporin, Granulozyte Colony Stimulating Factor (GCSF), Alpha-1 Proteinase Inhibitor, Elcatonin, Granulocyte Makrophage Colony Stimulating Factor (GMCSF), Wachstumshormone, menschliches Wachstumshormon (HGH), Growth hormone releasing hormone (GHRH), Heparin, niedermolekulares Heparin (LMWH), Interferon alpha, Interferon beta, Interferon gamma, Interleukin-2, Luteinizing hormone releasing hormone (LHRH), Somatostatin, Somatostatin-Analoge einsschließlich Octreotide, Vasopressinanaloge, Follikel stimulierendes Hormon (FSH), Insulin like growth factor, Insulintropin, Interleukin-1 Rezeptorantagonist, Interleukin-3, Interleukin-4, Interleukin-6, Macrophage colony stimulating Factor (M-CSF), Nerven-Wachstumsfaktor, Parathyroidhormon (PTH), Thymosin alpha 1, IIb/IIIa Inhibitor, Alpha-1 Antitrypsin, Antikörper gegen respiratorisch syncytischen Virus, Cystic fibrosis transmembrane regulator gene (CFTR), Desoxyribonuclease (Dnase), Bactericide, permeability increasing protein (BPI), anti-CMV Anikörper, Interleukin-1 Rezeptor, Retinol, Retinyl-Ester, Tocopherole und deren Ester, Tocotrienole und deren Ester, Carotinoide insbesondere Beta-Carotin und andere natürliche und synthetische Antioxidantien, Retinol-Säuren, Pentamidine, Albuterolsulfat, Metaproterenolsulfat, Beclomethasondiprepionat, Triamcinolonacetamid, Budesonidacetonide, Ipratropiumbromid, Flunisolide, Fluticasone, Cromolynsodium, Ergotamintartrat und die Analogen, Agonisten und Antagonisten der oben genannten Stoffe. Aktive Wirkstoffe können weiterhin Nukleinsäuren in Form von reinen Nukleinsäuremolekülen, viralen Vektoren, assoziierten viralen Partikeln, Nukleinsäuren, die mit Lipiden oder einem Lipide beinhaltenden Material assoziiert oder darin enthalten sind, Plasmid-DNA oder -RNA oder andere Konstrukte aus Nukleinsäuren sein, die geeignet sind für die Zell-Transfection oder -Transformation, besonders bei Zellen der alveolären Region der Lunge. Der aktive Wirkstoff kann in verschiedenen Formen vorliegen, wie lösliche oder unlösliche geladene oder ungeladene Moleküle, Komponenten molekularer Komplexe oder pharmakologisch akzeptierte Hilfsstoffe. Der aktive Wirkstoff kann bestehen aus natürlich vorkommenden Molekülen, oder deren rekombinanter Produktion, oder die Moleküle können Analoge der natürlich vorkommenden oder rekombinant erzeugten aktiven Wirkstoffe sein, bei denen eine oder mehrere Aminosäuren addiert oder entfernt wurden. Weiterhin kann der aktive Wirkstoff abgeschwächten Lebendimpfstoff oder abgetötete Viren für den Impfstoffgebrauch enthalten. Im Falle des Wirkstoffes Insulin sind natürlich extrahiertes menschliches Insulin, rekombinantes menschliches Insulin, aus Rindern und/oder Schweinen extrahiertes Insulin, rekombinantes Schweine-oder Rinderinsulin und Mischungen der oben genannten Insuline eingeschlossen. Das Insulin kann in reiner, also in substanziell gereinigter Form vorliegen, kann aber auch Auszüge wie kommerziell üblich enthalten. Im Ausdruck "Insulin" sind auch Insulin-Analoge enthalten, bei denen eine oder mehrere der Aminosäuren des natürlich vorkommenden oder rekombinanten Insulins addiert oder entfernt wurden. Besonders ist die erfindungsgemäße Inhalationsvorrichtung für die Verabreichung von Vitamin A bzw. von Vitamin A-Ester und Retinsäure bzw. Retinsäure-Ester geeignet, auch in Kombination mit natürlichen und synthetischen Antioxidantien.

Im folgenden wird die Erfindung unter Bezugnahme auf die beigefügten Zeichnungen näher erläutert. Es zeigen:
- Figur 1: eine Querschnittsansicht einer ersten erfindungsgemäßen Ausführungsform eines erfindungsgemäßen regelbaren Ventils zum Einsatz in einer Inhalationsvorrichtung im montierten Zustand;
- Figur 2: eine Querschnittsansicht des regelbaren Ventils gemäß Figur 1 als Explosionsdarstellung;
- Figur 3a: eine Querschnittsansicht eines Gehäuses des in Figuren 1 und 2 dargestellten regelbaren Ventils;
- Figur 3b: eine Seitenansicht von rechts auf das Gehäuse gemäß Figur 3a;
- Figur 3c: eine Seitenansicht von links auf das Gehäuse gemäß Figur 3a;
- Figur 4a: eine Vorderansicht einer ersten Komponente eines Stellkolbens zur Verwendung im regelbaren Ventil gemäß Figuren 1 und 2;
- Figur 4b: eine Seitenansicht von rechts auf die Stellkolbenkomponente gemäß Figur 4a;
- Figur 4c: eine Seitenansicht von links auf die Stellkolbenkomponente gemäß Figur 4a;
- Figur 4d: eine Querschnittsansicht durch die Stellkolbenkomponente gemäß Figur 4a;
- Figur 4e: eine Vorderansicht einer zweiten Stellkolbenkomponente;
- Figur 4f: eine Seitenansicht von rechts auf die zweite Stellkolbenkomponente gemäß Figur 4e;
- Figur 4g: eine Seitenansicht von links auf die zweite Stellkolbenkomponente gemäß Figur 4e;
- Figur 4h: eine Querschnittsansicht entlange der Linie IVh - IVh von Figur 4g;
- Figur 4i: eine Querschnittsansicht entlang der Linie IVi - IVi von Figur 4f;
- Figur 4k: eine Vorderansicht eines aus der ersten und zweiten Stellkolbenkomponente zusammengesetzten Stellkolben;
- Figur 4l: eine Querschnittsansicht durch den zusammengesetzten Stellkolben gemäß Figur 4k;
- Figur 5a: eine Vorderansicht auf eine im regelbaren Ventil gemäß Figuren 1 und 2 verwendete Stellschraube;
- Figur 5b: eine Querschnittsansicht durch die Stellschraube gemäß Figur 5a;
- Figur 5c: eine Seitenansicht von links auf die Stellschraube gemäß Figur 5a;
- Figur 6a: eine Seitenansicht auf eine Membran bzw. Elastomerscheibe zum Einsatz im regelbaren Ventil gemäß Figuren 1 und 2;
- Figur 6b: eine Querschnittsansicht durch die Membran bzw. Elastomerscheibe gemäß Figur 6a;
- Figur 7a: eine Seitenansicht auf eine im regelbaren Ventil gemäß Figuren 1 und 2 verwendbare Druckplatte;
- Figur 7b: eine Vorderansicht der Druckplatte gemäß Figur 7a;
- Figur 8a: eine Seitenansicht auf eine im regelbaren Ventil gemäß Figuren 1 und 2 verwendbare Verschlussschraube;
- Figur 8b: eine Vorderansicht auf die Verschlussschraube gemäß Figur 8a;
- Figur 9a: eine Vorderansicht auf das Gehäuse mit Stellmechanismus gemäß den Figuren 1 und 2;
- Figur 9b: eine Seitenansicht von links auf das Gehäuse mit Stellmechanismus gemäß Figur 9a;
- Figur 10: eine Querschnittsansicht einer zweiten Ausführungsform eines regelbaren Ventils mit stufenweiser Einstellbarkeit;
- Figur 11: eine Querschnittsansicht auf das regelbare Ventil gemäß Figur 10 in Explosionsdarstellung;
- Figur 12a: eine Querschnittsansicht durch das Gehäuse des regelbaren Ventils gemäß Figuren 10 und 11;
- Figur 12b: eine Querschnittsansicht durch das Gehäuse gemäß Figur 12a entlang der Linie XIIb - XIIb;
- Figur 12c: eine Seitenansicht von links auf das Gehäuse gemäß Figur 12a;
- Figur 12d: eine Vorderansicht auf das Gehäuse gemäß Figur 12a;
- Figur 13a: eine Querschnittsansicht auf die Stellschraube für das regelbare Ventil gemäß Figuren 10 und 11;
- Figur 13b: eine Querschnittsansicht entlang der Linie XIIIb - XIIIb von Figur 13a; und
- Figur 13c: eine Seitenansicht von links auf die Stellschraube gemäß Fig. 13a.

Das erfindungsgemäße regelbare Ventil 2 wird nachfolgend in Kombination mit einer Inhalationsvorrichtung beschrieben, die insbesondere ein (nicht dargestelltes) Behältnis für ein vorbestimmtes Aerosolvolumen, eine (nicht dargestellte) Einrichtung zum Ein- oder Ausbringen von Aerosol aus einem Aerosolspender in das Behältnis und eine Steuereinrichtung aufweist, die den Inhalationsfluss während der gesamten Inhalation des Aerosols im Wesentlichen konstant hält. Die Steuereinrichtung besteht im wesentlichen aus dem regelbaren Ventil 2.

Alternativ weist die Inhalationsvorrichtung einen Spacer auf, oder ist beispielsweise ein MDI (metered dose inhaler), oder DPI (dry powder inhaler), oder Vernebler (Ultraschall oder Druckluft). Der Begriff "Inhalationsvorrichtung" umfasst ferner Trainingsgeräte oder Diagnosegeräte.

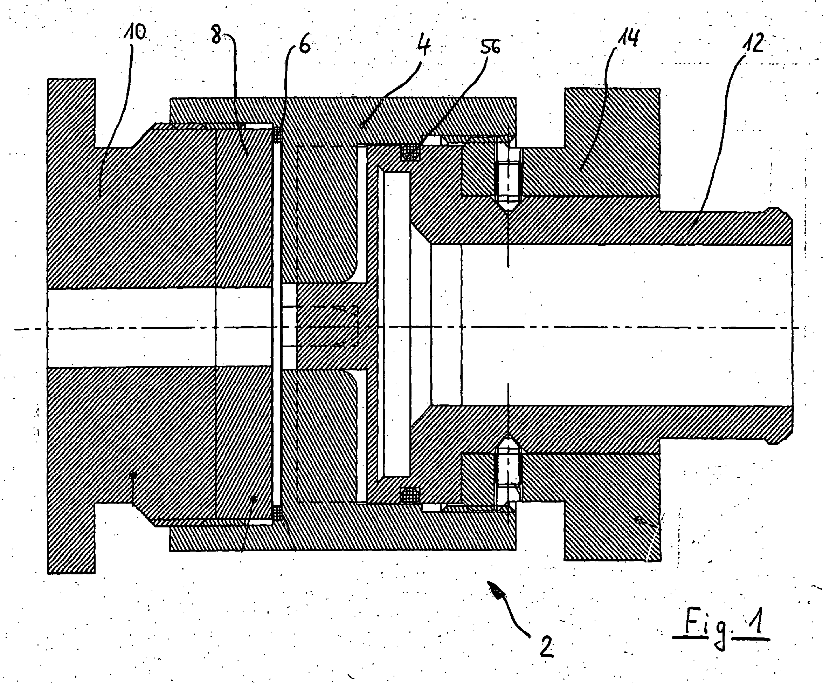

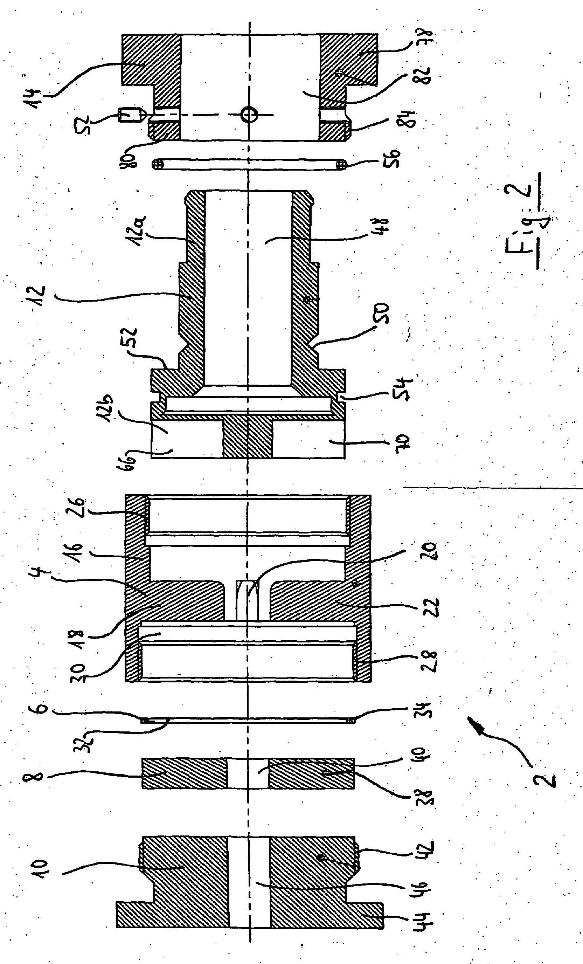

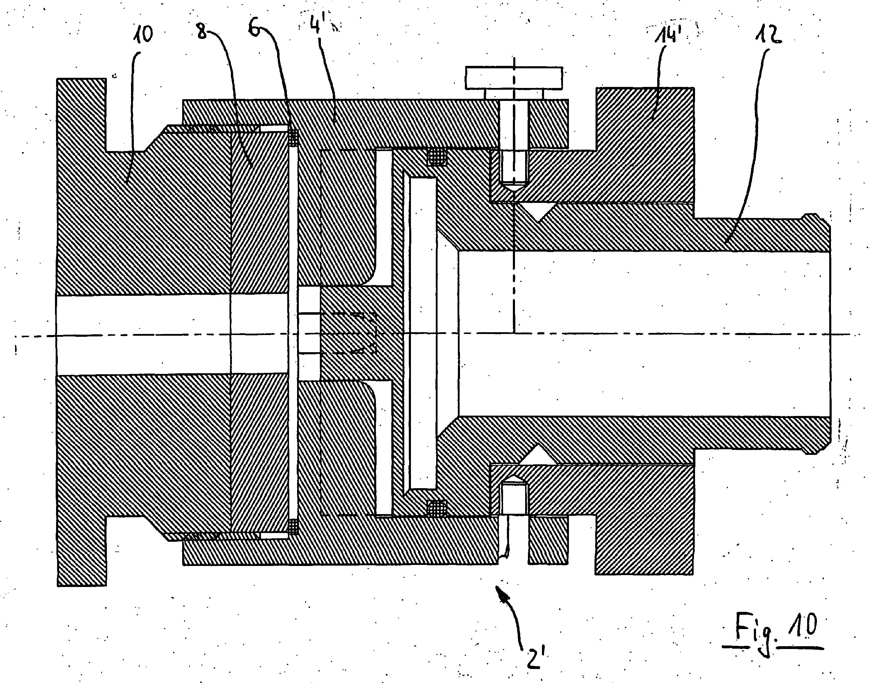

Gemäß der in den Figuren 1-9 dargestellten Ausführungsform ist das erfindungsgemäße regelbare Ventil 2 in Form eines stufenlos einstellbaren Flussbegrenzers ausgebildet.

Das regelbare Ventil 2 weist im wesentlichen ein Gehäuse 4, eine Membran bzw. Elastomerscheibe 6, eine Druckplatte 8, ein Verschlusselement 10, einen axial beweglich geführten Stellkolben 12 und eine Stellschraube 14 zum Einstellen der Durchflussmenge durch das regelbare Ventil 2 auf.

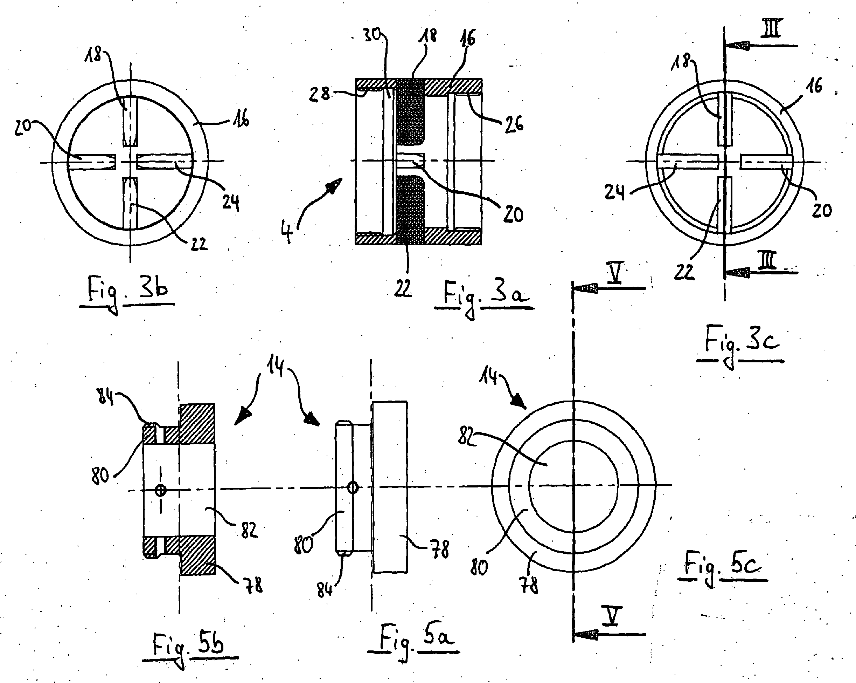

Das Gehäuse 4 des regelbaren Ventils 2 ist in den Figuren 3a, 3b und 3c näher gezeigt. Wie insbesondere in Figur 3a ersichtlich, ist das Gehäuse 4 im wesentlichen zylinderrohrförmig mit einer Rohrwand 16 ausgebildet. Das Gehäuse 4 weist mehrere, vorzugsweise vier, sternförmig angeordnete Stege 18, 20, 22, 24 auf, von denen ein Steg 24 länger ist als die übrigen Stege. In der in den Figuren 3a, 3b und 3c dargestellten Ausführungsform des Gehäuses 4 sind die Stege 18, 20, 22, 24 in die rohrförmige Wandung 16 des Gehäuses 4 eingesetzt und damit verklebt. Alternativ könnte das Gehäuse jedoch auch einstückig bzw. aus einem Teil hergestellt sein.

Das Gehäuse 4 weist in Axialrichtung einerseits der Stege 18, 20, 22, 24 ein Innengewinde 26 zur verstellbaren Aufnahme des Stellkolbens 12 mittels der Stellschraube 14 auf. Auf der axial gegenüberliegenden Seite der Stege 18, 20, 22, 24 weist das Gehäuse 4 ebenfalls ein Innengewinde 28 zur Aufnahme des Verschlusselements 10 auf, das vorzugsweise als Verschlussschraube ausgebildet ist. Ferner ist auf der gleichen Seite wie das Gewinde 28 eine Nut 30 zur Aufnahme der Membran bzw. Elastomerscheibe 6 vorgesehen.

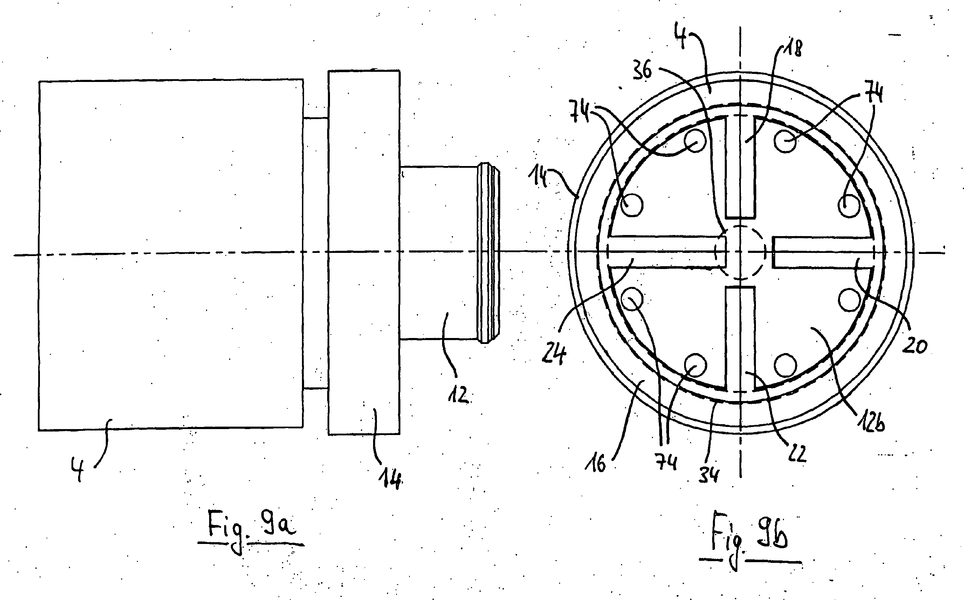

Die Membran bzw. Elastomerscheibe 6 ist in den Figuren 6a und 6b näher gezeigt und weist vorzugsweise eine scheibenförmige Gestalt mit einem Scheibeninneren 32 sowie einem Kragen 34 auf. Im Zentrum der Membran 6 ist eine Öffnung 36 vorgesehen.

Die Druckplatte 8 ist in den Figuren 7a und 7b näher gezeigt. Die Druckplatte ist im wesentlichen eine Zylinderplatte 38 mit einer zentralen Öffnung 40. Sie ist dazu vorgesehen, die Membran 6 im Gehäuse 4 in der Nut 30 zu halten, ohne dass dabei bei der Montage oder im Betrieb eine Gefahr der Beschädigung der Membran 6 besteht.

Die Membran 6 sowie die Druckplatte 8 werden vorzugsweise durch die Verschlussschraube 10 fixiert, die mittels eines in das Gewinde 28 am Gehäuse 4 eingreifenden Außengewindes 42 in das Gehäuse 4 einschraubbar ist. Die Verschlussschraube 10 weist ferner einen Knauf 44 zum Betätigen der Verschlussschraube 10 auf. Ferner ist in der Verschlussschraube 10 eine zentrale Durchgangsöffnung 46 vorgesehen, die sowohl mit der Durchgangsöffnung 40, der Druckplatte 8 als auch mit der Durchgangsöffnung 36 der Membran 6 im Wesentlichen fluchtet.

Im montierten Zustand wird die Membran 6 mittels der Druckplatte 8 und der Verschlussschraube 10 in eine vorbestimmte und festgelegte Position gedrängt, in der die Membran mit ihrem Scheibeninneren 32 auf den Stegen 18, 20, 22, 24 des Gehäuses 4 aufliegt. Der Kragen 34 der Membran 6 erstreckt sich dabei in Richtung von den Stegen 18, 20, 22, 24 weg. Dies ist in Figur 1 anschaulich gezeigt.

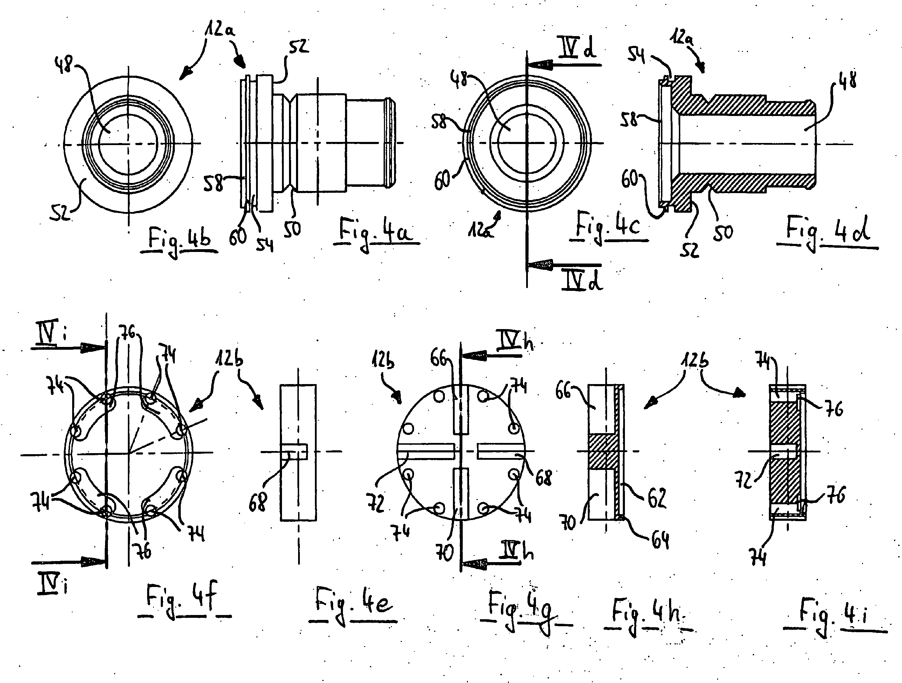

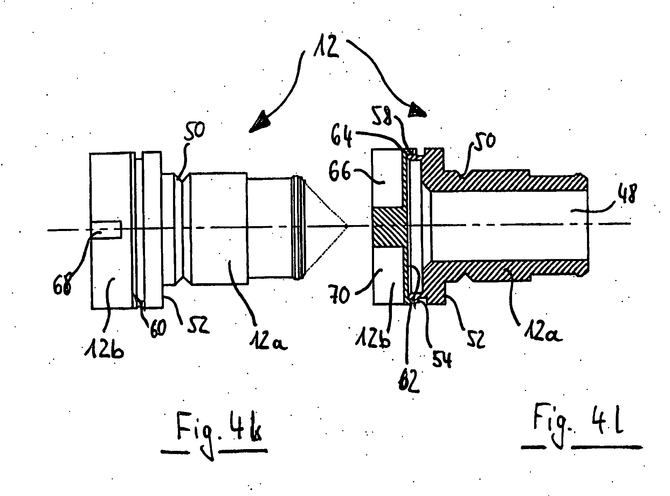

Der Stellkolben 12 des regelbaren Ventils 2 ist in den Figuren 4a-4l näher dargestellt. Der Stellkolben 12 weist gemäß den Darstellungen in Figuren 4a-4d eine erste Stellkolbenkomponente 12a sowie gemäß den Darstellungen in Figuren 4e-4i eine zweite Stellkolbenkomponente 12b auf. Die erste Stellkolbenkomponente 12a ist in Figur 4a in Vorderansicht und in Figuren 4b und 4c jeweils in Seitenansicht von rechts bzw. links gezeigt. Figur 4d stellt eine Querschnittsansicht durch die erste Stellkolbenkomponente 12a dar.

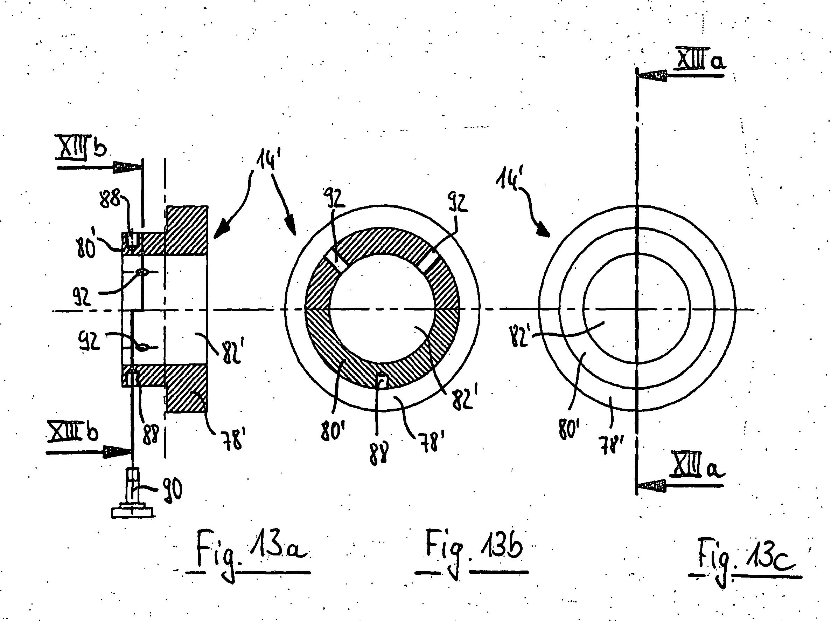

Die erste Stellkolbenkomponente 12a weist eine Durchgangsöffnung bzw. einen zentralen Strömungskanal 48 auf, der insbesondere in Figur 12d gut erkennbar ist. Des weiteren weist die erste Stellkolbenkomponente 12a eine umlaufende Nut 50 zur Aufnahme einer oder mehrerer Feststellschrauben 52, zum Beispiel von Madenschrauben, auf, mit denen die Stellschraube 14 am Stellkolben 12 in Axialrichtung fixiert wird. Außerdem weist die erste Stellkolbenkomponente 12a einen Anschlag 52 für die Stellschraube 14 auf. Des weiteren weist die erste Stellkolbenkomponente 12a eine umlaufende Nut 54 zur Aufnahme eines Dichtrings 56 sowie einen Vorsprung 58 und einen Anschlag 60 zur Aufnahme der zweiten Stellkolbenkomponente 12b auf.

Die zweite Stellkolbenkomponente 12b weist ihrerseits, wie insbesondere in Figur 4h gezeigt, eine Vertiefung 62 zur Aufnahme des Vorsprungs 58 sowie einen Kragen 64 auf, der mit dem Anschlag 60 in Kontakt bringbar ist. In diesem Bereich werden die erste und die zweite Stellkolbenkomponente 12a, 12b vorzugsweise miteinander verklebt, so dass sie als einheitliches Bauteil vorliegen.

Wie in Figur 4e gezeigt, besteht die zweite Stellkolbenkomponente 12b aus einem im Wesentlichen zylindrischen Element, das gemäß der in Figur 4g dargestellten Seitenansicht von links vier Ausnehmungen 66, 68, 70, 72 aufweist, die entsprechend bzw. korrespondierend zu den Stegen 18, 20, 22, 24 im Gehäuse 4 ausgebildet sind. Dementsprechend ist auch eine der Ausnehmungen 72 länger als die anderen Ausnehmungen 66, 68, 70. Beim Einsetzen des Stellkolbens 12 in das Gehäuse 4 nehmen die Ausnehmungen 66,68,70,72 die Stege 18, 20, 22, 24 entsprechend der jeweiligen Axialposition des Stellkolbens 12 relativ zum Gehäuse 4 auf. Dies ist in Figur 1 anschaulich gezeigt.

Im Bereich zwischen zwei benachbarten Ausnehmungen weist die zweite Stellschraubenkomponente 12b, wie beispielsweise in den Figuren 4f und 4g gezeigt mehrere Öffnungen 74 auf. Diese Öffnungen 74 münden in einen in den Figuren 4f und 4i näher gezeigten flachen Kanal 76, der jeweils zwei benachbarte und zwischen zwei Stegen liegende Öffnungen 74 strömungsmäßig miteinander verbindet. Die Strömungskanäle 76 der zweiten Stellkolbenkomponente 12b münden wiederum in den Strömungskanal 48 der ersten Stellkolbenkomponente 12a.

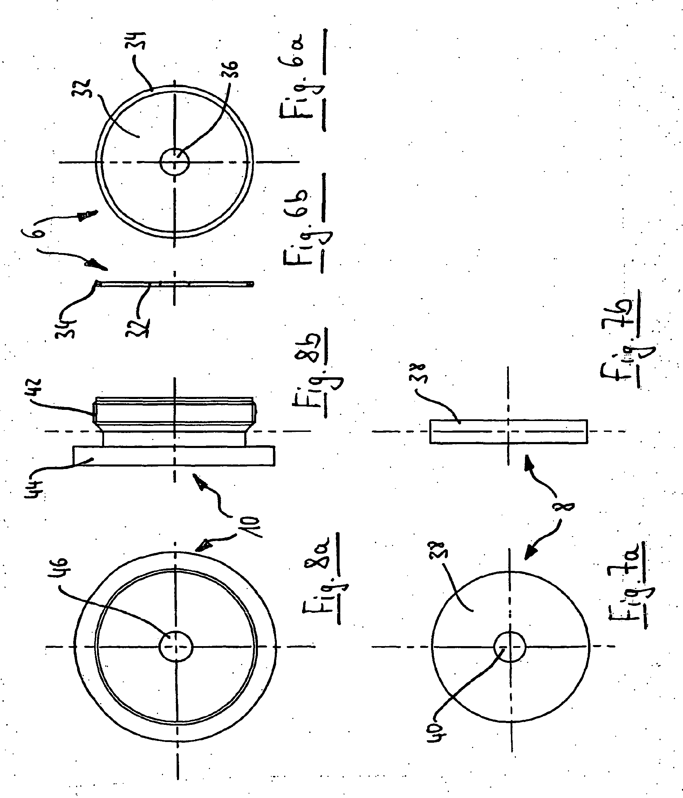

In den Figuren 5a, 5b und 5c ist die Stellschraube 14 näher gezeigt. Die Stellschraube 14 weist einen Griffabschnitt 78, einen Eingriffsabschnitt 80 sowie eine zentrale Durchgangsöffnung 82 auf. Der Eingriffsabschnitt 80 ist mit einem Außengewinde 84 versehen, das mit dem Gewinde 26 des Gehäuses 4 in Eingriff bringbar ist, um den Stellkolben 12 im Gehäuse axial zu positionieren. Die Verbindung zwischen der Stellschraube 14 und dem Stellkolben 12 erfolgt mittels der Madenschraube(n) 52, die in die Nut 50 am Stellkolben eingreift bzw. eingreifen, um eine axiale Verschiebung der beiden Komponenten relativ zueinander zu unterbinden. Hierzu sind vorzugsweise um den Umfang der Stellschraube verteilt mehrere Madenschrauben 52, vorzugsweise 4 um jeweils 90 Grad versetzte Madenschrauben, vorgesehen.

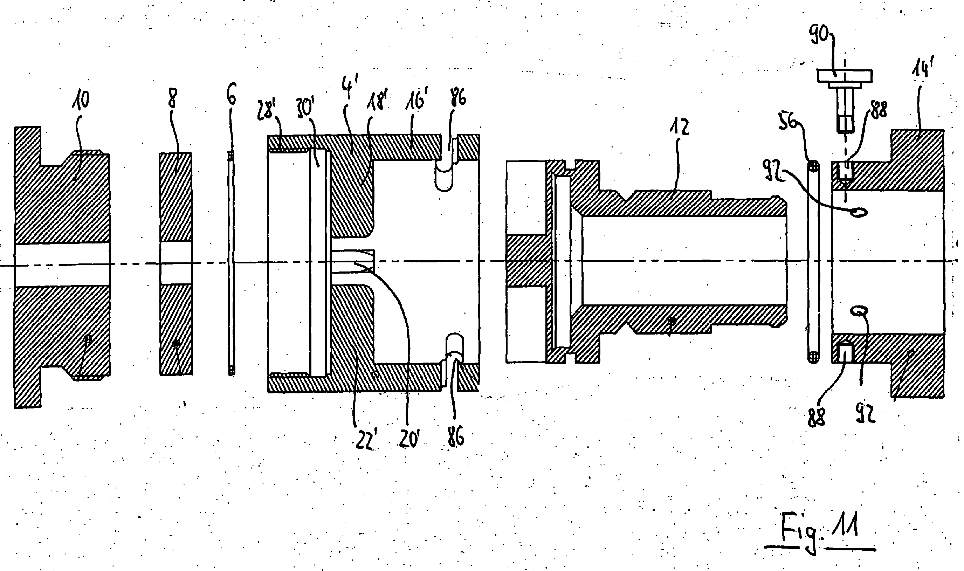

In den Figuren 9a und 9b ist das Gehäuse 4 und der Stellmechanismus 12, 14 nochmals in montiertem Zustand (ungeschnitten) gezeigt. Insbesondere in der in Figur 9b gezeigten Seitenansicht von links, in der die Verschlussschraube 10 und Druckplatte 8 weggelassen sind, ist gut zu erkennen, dass die Wand 16 des Gehäuses 4 das regelbare Ventil nach radial außen begrenzt, während die vier Stege 18, 20, 22, 24 im wesentlichen sternförmig nach radial innen ragen. Die zweite Stellkolbenkomponente 12b ist in der Ansicht gemäß Figur 9b ebenfalls zu erkennen, wobei insbesondere die Öffnungen 74 davon zu sehen sind. Die Membran 6 ist in Figur 9b transparent dargestellt und liegt auf den Stegen 18, 20, 22, 24 sowie in der Nut 30 auf. Der Kragen 34 der Membran ist in Figur 9b gestrichelt eingetragen. Ebenfalls gestrichelt eingetragen ist die zentrale Öffnung 36 der Membran 6.

Die vorstehend unter Bezugnahme auf die Figuren 1-9 beschriebene erste Ausführungsform des erfindungsgemäßen regelbaren Ventils 2 ermöglicht einen stufenlos variabel einstellbaren Volumenstrom durch das Ventil.

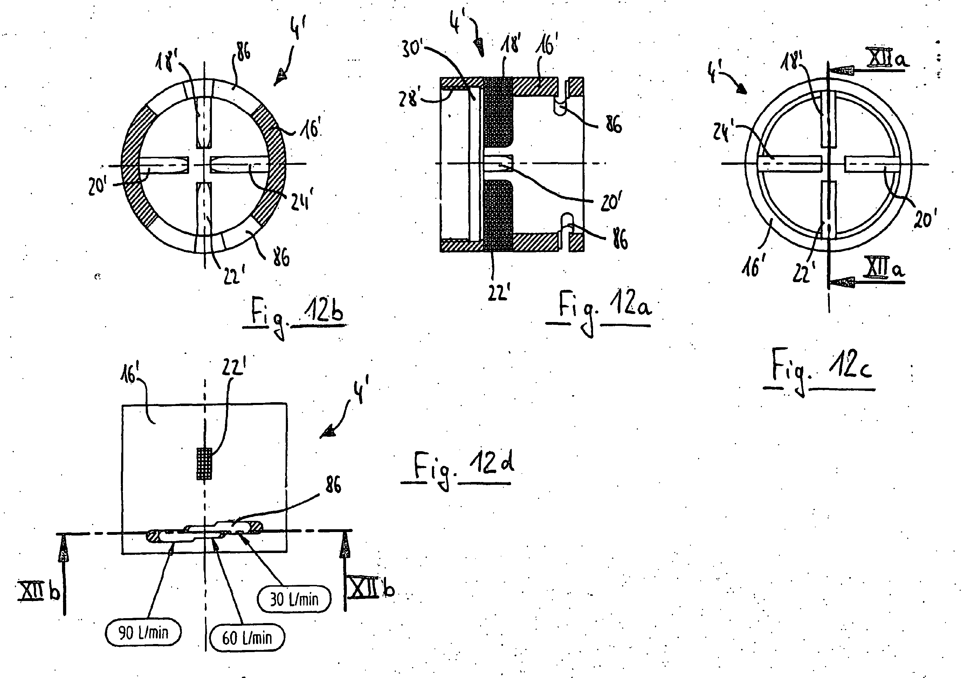

In den Figuren 10-13 ist eine zweite erfindungsgemäße Ausführungsform eines regelbaren Ventils 2' dargestellt, bei der der durch das Ventil strömende Volumenstrom stufenweise, zum Beispiel in drei Stufen, einstellbar ist. In Figur 10 ist das regelbare Ventil 2' im Querschnitt und montierten Zustand dargestellt. Das Ventil 2' weist ein Gehäuse 4', eine Membran bzw. Elastomerscheibe 6, eine Druckplatte 8, eine Verschlussschraube 10, einen Stellkolben 12 sowie eine Stellschraube 14' auf. Soweit nicht explizit beschrieben korrespondieren die Elemente des regelbaren Ventils 2' der zweiten Ausführungsform mit denen der ersten Ausführungsform.

Das Gehäuse 4' des regelbaren Ventils gemäß der zweiten Ausführungsform ist in Figuren 12a, 12b, 12c und 12d näher gezeigt. Das Gehäuse 4' ist ebenfalls im wesentlichen rohrförmig ausgebildet und weist eine Wand 16' sowie vier Stege 18', 20', 22', 24' auf. Ferner ist ein Gewinde 28' zur Aufnahme der Verschlussschraube 10 sowie eine Nut 30' zur Aufnahme der Membran bzw. Elastomerscheibe 6 vorgesehen.

Auf der der Membranaufnahmenut 30' gegenüberliegenden Seite der Stege ist das Gehäuse 4' jedoch nicht mit einem Gewinde zur Aufnahme einer Stellschraube wie in der ersten Ausführungsform ausgebildet, sondern vielmehr mit einer Kulissenführung in Form einer oder mehrerer in Axialrichtung abgestufter Aussparung(en) 86, wie dies insbesondere in Figur 12d näher gezeigt ist. Durch die stufenförmige Ausgestaltung der Aussparung 86 ist der Stellkolben 12 in drei verschiedene Axialpositionen einstellbar, nämlich beispielsweise für ein Volumendurchsatz von 30 1/min, 60 1/min oder 90 1/min, wie in Figur 12d schematisch gezeigt.

Der Stellkolben 12 entspricht im wesentlichen dem Stellkolben, der in Zusammenhang mit der ersten Ausführungsform beschrieben wurde und ist im Gehäuse 4' axial beweglich und dichtend geführt.

In den Figuren 13a, 13b und 13c ist die Stellschraube 14' für das regelbare Ventil 2' der zweiten Ausführungsform näher gezeigt. Ähnlich wie im Fall der ersten Ausführungsform weist die Stellschraube 14' einen Betätigungsabschnitt 78', sowie einen Eingriffsabschnitt 80' und einen zentralen Strömungskanal 82' auf. Jedoch ist die Stellschraube 14' nicht mit einem Außengewinde zur Verbindung mit dem Gehäuse versehen, sondern weist vielmehr radial vorgesehene Gewindesacklöcher 88 auf, in die Kulissenführungsschrauben 90 durch die Aussparung(en) 86 am Gehäuse 4' einschraubbar sind. Daneben weist die Stellschraube 14' ähnlich wie im Fall der ersten Ausführungsform Gewindebohrungen 92 auf, die zur Aufnahme von Madenschrauben 52 vorgesehen sind, um die Stellschraube 14' bezüglich des Stellkolbens 12 axial zu fixieren.

## Patentansprüche

1. Inhalationsvorrichtung mit einer Steuereinrichtung, die den Inhalationsfluss während der gesamten Inhalation des Aerosols im Wesentlichen konstant hält; wobei die Steuereinrichtung ein regelbares Ventil (2; 2') aufweist, das ein Gehäuse (4; 4'), ein Verschlusselement (10) und eine Membran (6) hat, **dadurch gekennzeichnet, dass** das Ventil (2;2') eine optionale Druckplatte (8), einen Stellkolben (12) und eine Stellschraube (14; 14') zum Einstellen der Durchflussmenge durch das regelbare ventil (2) aufweist.

2. Inhalationsvorrichtung nach Anspruch 1, ferner mit einem Behältnis für ein vorbestimmtes Aerosolvolumen, einer Einrichtung zum Einbringen von Aerosol aus einem Aerosolspender in das Behältnis und einem Dosieraerosolanschluss, einem Vernebler und/oder einer Inhalationshilfe.

3. Inhalationsvorrichtung nach Anspruch 1 oder 2, wobei das regelbare Ventil in Form eines stufenlos variablen Flussbegrenzers (2) ausgebildet ist.

4. Inhalationsvorrichtung nach Anspruch 1 oder 2, wobei das regelbare Ventil in Form eines in mehreren Stufen einstellbaren Flussbegrenzers (2) ausgebildet ist.

5. Inhalationsvorrichtung nach einem der Ansprüche 1 bis 4, wobei das Gehäuse (4;'4') im Wesentlichen rohrförmig ausgebildet ist und mehrere, vorzugsweise vier, sternförmig angeordnete Stege (18, 20, 22, 24; 18', 20', 22', 24') aufweist, von denen ein Steg (24; 24') länger ist als die anderen.

6. Inhalationsvorrichtung nach Anspruch 5, wobei das Gehäuse (4; 4') einerseits der Stege zur verstellbaren Aufnahme des Stellkolbens (12) und auf der gegenüberliegenden Seite zur Aufnahme der Membran (6), der Druckplatte (8) und des Verschlusselements (10) ausgebildet ist.

7. Inhalationsvorrichtung nach einem der Ansprüche 1 bis 6, wobei die Membran (6) mittels der Druckplatte (8) und des Verschlusselements (10) gegen die Stege (18, 20, 22, 24; 18', 20', 22', 24') des Gehäuses (4; 4') gedrängt wird, so dass zwischen der Membran (6) und dem Stellkolben (12) ein Volumenraum gebildet ist.

8. Inhalationsvorrichtung nach einem der Ansprüche 1 bis 7, wobei die Membran (6) scheibenförmig ausgebildet ist und an ihrem Rand einen Kragen (34) aufweist.

9. Inhalationsvorrichtung nach einem der Ansprüche 5 bis 8, wobei der Stellkolben (12) an seiner zur Membran (6) gerichteten Stirnseite mehrere, vorzugsweise vier, entsprechend den Stegen (18, 20, 22, 24; 18', 20', 22', 24') des Gehäuses (4; 4') sternförmig ausgebildete Ausnehmungen (66, 68, 70, 72) aufweist, wobei eine Ausnehmung (72) länger ist als die anderen.

10. Inhalationsvorrichtung nach einem der Ansprüche 1 bis 9, wobei der Stellkolben (12) dichtend im Gehäuse (4; 4') aufgenommen ist.

11. Inhalations vorrichtung nach einem der Ansprüche 1 bis 10, wobei die Stellschraube (14; 14') am Stellkolben (12) in Axialrichtung fixierbar ist und der Stellkolben (12) mittels der Stellschraube (14; 14') relativ zum Gehäuse (4; 4') in Axialrichtung einstellbar ist.

12. Inhalationsvorrichtung nach einem der Ansprüche 5 bis 11, wobei die Stellschraube (14; 14') im Bereich zwischen zwei benachbarten Ausnehmungen mindestens eine, vorzugsweise zwei, Öffnung(en) (74) aufweist.

13. Inhalations vorrichtung nach Anspruch 12, wobei jeweils zwei Öffnungen (74) zwischen zwei benachbarten Ausnehmungen (66, 68, 70, 72) vorgesehen sind, wobei die Öffnungen (74) auf einer gemeinsamen Umfangslinie liegen und jeweils um etwa 45° voneinander beabstandet sind.

14. Inhalationsvorrichtung nach einem der Ansprüche 5 bis 13, wobei im Gehäuse (4; 4'), der Membran (6), der Druckplatte (8), dem Verschlusselement (10) und dem Stellkolben (12) zumindest abschnittsweise ein zentraler Strömungskanal vorgesehen ist.

15. Inhalationsvorrichtung nach einem der Ansprüche 4 bis 14, wobei die Stellschraube (14) in das Gehäuse (4) eingeschraubt ist.

16. Inhalationsvorrichtung nach einem der Ansprüche 4 bis 14, wobei die Stellschraube (14') mittels einer Kulissenführung (86, 90) mit dem Gehäuse (4') verbunden ist, um den Stellkolben (12) axial zu führen.

17. Regelbares Ventil, insbesondere zur Verwendung in einer Inhalationsvorrichtung, mit:
einem Gehäuse (4; 4'), einer Membran (6), einem Verschlusselement (10), wobei das Gehäuse (4; 4') im wesentlichen rohrförmig ausgebildet ist und mehrere, vorzugsweise vier, sternförmig angeordnete Stege (18, 20, 22, 24; 18', 20', 22', 24') aufweist, **dadurch gekennzeichnet, dass** das Ventil eine optionale Druckplatte (8), einen Stellkolben (12) und eine Stellschraube (14; 14') zum Einstellen der Durchflussmenge durch das regelbare Ventil aufweist und ein Steg (24; 24') länger ist als die anderen, wobei das Gehäuse (4; 4') einerseits der Stege zur verstellbaren Aufnahme des Stellkolbens (12) und auf der gegenüberliegenden Seite zur Aufnahme der Membran (6), der optionalen Druckplatte (8) und des Verschlusselements (10) ausgebildet ist.

18. Regelbares Ventil nach Anspruch 17, wobei die Membran (6) mittels der Druckplatte (8) des Verschlusselements (10) gegen die Stege (18, 20, 22, 24; 18', 20', 22', 24') des Gehäuses (4; 4') gedrängt wird, so dass zwischen der Membran (6) und dem Stellkolben (12) ein Volumenraum gebildet ist.

19. Regelbares Ventil nach Anspruch 17 oder 18, wobei die Membran (6) scheibenförmig ausgebildet ist und an ihrem Rand einen Kragen (34) aufweist.

20. Regelbares Ventil nach einem der Ansprüche 17 bis 19, wobei der Stellkolben (12) an seiner zur Membran (6) gerichteten Stirnseite mehrere, vorzugsweise vier, entsprechend den Stegen (18, 20, 22, 24; 18', 20', 22', 24') des Gehäuses (4; 4') sternförmig ausgebildete Ausnehmungen (66, 68, 70, 72) aufweist, wobei eine Ausnehmung (72) länger ist als die anderen.

21. Regelbares Ventil nach einem der Ansprüche 17 bis 20, wobei der Stellkolben (12) dichtend im Gehäuse (4; 4') aufgenommen ist.

22. Regelbares Ventil nach einem der Ansprüche 17 bis 21, wobei die Stellschraube (14; 14') am Stellkolben (12) in Axialrichtung fixierbar ist und der Stellkolben (12) mittels der Stellschraube (14; 14') relativ zum Gehäuse (4; 4') in Axialrichtung einstellbar ist.

23. Regelbares Ventil nach einem der Ansprüche 17 bis 22, wobei die Stellschraube (14, 14') im Bereich zwischen zwei benachbarten Ausnehmungen mindestens eine, vorzugsweise zwei, Öffnung(en) (74) aufweist.

24. Regelbares Ventil nach Anspruch 23, wobei jeweils zwei Öffnungen (74) zwischen zwei benachbarten Ausnehmungen (66. 68, 70, 72) vorgesehen sind, wobei die Öffnungen (74) auf einer gemeinsamen Umfangslinie liegen und jeweils um etwa 45° voneinander beabstandet sind.

25. Regelbares Ventil nach einem der Ansprüche 17 bis 24, wobei im Gehäuse (4; 4'), der Membran (6), der Druckplatte (8), dem Verschlusselement (10) und dem Stellkolben (12) zumindest abschnittsweise ein zentraler Strömungskanal vorgesehen ist.

26. Regelbares Ventil nach einem der Ansprüche 17 bis 25, wobei die Stellschraube (14) in das Gehäuse (4) eingeschraubt ist.

27. Regelbares Ventil nach einem der Ansprüche 17 bis 25, wobei die Stellschraube (14') mittels einer Kulissenführung (86, 90) mit dem Gehäuse (4') verbunden ist, um den Stellkolben (12) axial zu führen.

28. Regelbares Ventil nach einem der Anspruche 17. bis 27, das in Form eines stufenlos variablen Flussbegrenzers ausgebildet ist.

29. Regelbares Ventil nach einem der Ansprüche 17 bis 27, das in Form eines in mehreren Stufen einstellbaren Flussbegrenzers ausgebildet ist.

30. Regelbares Ventil nach einem der Ansprüche 17 bis 29, wobei das Ventil an eine Inhalationsvolumenbegrenzung angeschlossen ist.

31. Regelbares Ventil nach Anspruch 30, wobei die Volumenbegrenzung direkt oder indirekt über die Zeit erfolgt.

## Claims

1. An inhalation device comprising a control means that keeps the inhalation flow essentially constant during the entire aerosol inhalation, wherein the control means comprises a controllable valve (2; 2') having a housing (4; 4'), a closure element (10), and a membrane (6), **characterized in that** the valve (2; 2') comprises an optional pressure plate (8), a set piston (12) and an adjusting screw (14; 14') for adjusting the flow rate through the controllable valve (2).

2. The inhalation device according to claim 1, further comprising a container for a predetermined aerosol volume, means for disbursing an aerosol from an aerosol dispenser into the container as well as an aerosol dosing supply, a nebulizer and/or an inhalation aid.

3. The inhalation device according to claim 1 or 2, wherein the controllable valve is a continuously variable flow limiter (2).

4. The inhalation device according to claim 1 or 2, wherein the controllable valve is a flow limiter (2) that is adjustable in several steps.

5. The inhalation device according to any of claims 1 to 4, wherein the housing (4; 4') is essentially tubular and comprises a plurality of, preferably four, radially arranged webs (18, 20, 22, 24; 18', 20', 22', 24'), one web (24; 24') being longer than the others.

6. The inhalation device according to claim 5, wherein on the one side of the webs the housing (4; 4') is designed so as to adjustably receive the set piston (12) and on the opposite side so as to receive the membrane (6), the pressure plate (8) and the closure element (10).

7. The inhalation device according to any of claims 1 to 6, wherein the membrane (6) is forced against the webs (18, 20, 22, 24; 18', 20', 22', 24') of the housing (4; 4') by the pressure plate (8) and the closure element (10) so that a space is formed between the membrane (6) and the set piston (12).

8. The inhalation device according to any of claims 1 to 7, wherein the membrane (6) is disk-shaped and provided with a collar (34) at its edge.

9. The inhalation device according to any of claims 5 to 8, wherein the set piston (12) comprises a plurality of, preferably four, radially arranged recesses (66, 68, 70, 72) corresponding to the webs (18, 20, 22, 24; 18', 20', 22', 24') of the housing (4; 4') at its front end facing the membrane (6), one recess (72) being longer than the others.

10. The inhalation device according to any of claims 1 to 9, wherein the set piston (12) is sealingly received in the housing (4; 4').

11. The inhalation device according to any of claims 1 to 10, wherein the adjusting screw (14; 14') is axially fixable to the set piston (12) and the set piston (12) is axially adjustable with respect to the housing (4; 4') via the adjusting screw (14; 14').

12. The inhalation device according to any of claims 5 to 11, wherein the adjusting screw (14; 14') comprises one, preferably two, opening(s) (74) in the area between two adjoining recesses.

13. The inhalation device according to claim 12, wherein between each pair of adjoining recesses (66, 68, 70, 72) two openings (74) are provided which are situated on a common circumferential line and are spaced apart from each other by approx. 45°.

14. The inhalation device according to any of claims 5 to 13, wherein at least portions of the housing (4; 4'), the membrane (6), the pressure plate (8), the closure element (10) and the set piston (12) are provided with a central flow channel.

15. The inhalation device according to any of claims 4 to 14, wherein the adjusting screw (14) is screwed into the housing (4).

16. The inhalation device according to any of claims 4 to 14, wherein the adjusting screw (14') is connected to the housing (4') via a sliding block guide (86, 90) so as to axially guide the set piston (12).

17. A controllable valve, in particular for use in an inhalation device, comprising:
a housing (4; 4'), a membrane (6), a closure element (10), wherein the housing (4; 4') is essentially tubular and comprises a plurality of, preferably four, radially arranged webs (18, 20, 22, 24; 18', 20', 22', 24'), **characterized in that** the valve comprises an optional pressure plate (8), a set piston (12) and an adjusting screw (14; 14') for adjusting the flow rate through the controllable valve, and one web (24; 24') being longer than the others, wherein on one side of the webs the housing (4; 4') is designed so as to adjustably receive the set piston (12) and on the opposite side so as to receive the membrane (6), the optional pressure plate (8) and the closure element (10).

18. The controllable valve according to claim 17, wherein the membrane (6) is forced against the webs (18, 20, 22, 24; 18', 20', 22', 24') of the housing (4; 4') by means of the pressure plate (8) of the closure element (10) so that a space is formed between the membrane (6) and the set piston (12).

19. The controllable valve according to claim 17 or 18, wherein the membrane (6) is disk-shaped and is provided with a collar (34) at its edge.

20. The controllable valve according to any of claims 17 to 19, wherein at its front end facing the membrane (6) the set piston (12) comprises a plurality of, preferably four, radially arranged recesses (66, 68, 70, 72) corresponding to the webs (18, 20, 22, 24; 18', 20', 22', 24') of the housing (4; 4'), one recess (72) being longer than the others.

21. The controllable valve according to any of claims 17 to 20, wherein the set piston (12) is sealingly received within the housing (4; 4').

22. The controllable valve according to any of claims 17 to 21, wherein the adjusting screw (14; 14') is axially fixable to the set piston (12) and the set piston (12) is axially adjustable with respect to the housing (4; 4') by means of the adjusting screw (14; 14').

23. The controllable valve according to any of claims 17 to 22, wherein in the area between two adjoining recesses the adjusting screw (14, 14') is provided with at least one, preferably two opening(s) (74).

24. The controllable valve according to claim 23, wherein each pair of adjoining recesses (66, 68, 70, 72) is provided with two openings (74) which are situated on a common circumferential line and are spaced apart from each other by approx. 45°.

25. The controllable valve according to any of claims 17 to 24, wherein at least portions of the housing (4; 4'), the membrane (6), the pressure (8), the closure (10) and the set piston (12) are provided with a central flow channel.

26. A controllable valve according to any of claims 17 to 25, wherein the adjusting screw (14) is screwed into the housing (4).

27. The controllable valve according to any of claims 17 to 25, wherein the adjusting screw (14') is connected to the housing (4') by means of a sliding block guide (86, 90) so as to axially guide the set piston (12).

28. The controllable valve according to any of claims 17 to 27 in the form of a continuously variable flow limiter.

29. The controllable valve according to any of claims 17 to 27 in the form of a flow limiter that is adjustable in several steps.

30. The controllable valve according to any of claims 17 to 29, wherein the valve is connected to an inhalation flow rate limitation.

31. The controllable valve according to claim 30, wherein the flow rate limitation is directly or indirectly performed over time.

## Revendications

1. Dispositif d'inhalation muni d'un dispositif de commande qui maintient sensiblement constant le flux d'inhalation pendant la totalité de l'inhalation de l'aérosol, le dispositif de commande comprenant une valve réglable (2 ; 2') qui possède un boîtier (4 ; 4'), un élément obturateur (10) et une membrane (6), **caractérisé en ce que** la valve (2 ; 2') comprend une plaque de pression optionnelle (8), un piston de positionnement (12) et une vis de positionnement (14 ; 14') pour régler le débit quantitatif à travers la valve réglable (2).

2. Dispositif d'inhalation selon la revendication 1, comprenant en outre un réceptacle pour un volume prédéterminé d'aérosol, un dispositif pour introduire de l'aérosol depuis un distributeur d'aérosol dans le réceptacle et un raccord de dosage d'aérosol, un vaporisateur et/ou un auxiliaire d'inhalation.

3. Dispositif d'inhalation selon la revendication 1 ou 2, dans lequel la valve réglable est réalisée sous la forme d'un limiteur de flux (2) variable en continu.

4. Dispositif d'inhalation selon la revendication 1 ou 2, dans lequel la valve réglable est réalisée sous la forme d'un limiteur de flux (2) réglable à plusieurs étages.

5. Dispositif d'inhalation selon l'une des revendications 1 à 4, dans lequel le boîtier (4 ; 4') est réalisé sensiblement tubulaire et comprend plusieurs, de préférence quatre barrettes (18, 20, 22, 24 ; 18', 20', 22', 24') agencées en étoile dont une barrette (24 ; 24') est plus longue que les autres.

6. Dispositif d'inhalation selon la revendication 5, dans lequel, sur un côté des barrettes, le boîtier (4 ; 4') est réalisé pour le logement du piston de positionnement (12) avec possibilité de réglage et sur le côté opposé il est réalisé pour le logement de la membrane (6), de la plaque de pression (8) et de l'élément obturateur (10).

7. Dispositif d'inhalation selon l'une des revendications 1 à 6, dans lequel la membrane (6) est refoulée contre les barrettes (18, 20, 22, 24 ; 18', 20', 22', 24') du boîtier (4 ; 4') au moyen de la plaque de pression (8) et de l'élément obturateur (10), de sorte qu'il se forme un espace volumétrique entre la membrane (6) et le piston de positionnement (12).

8. Dispositif d'inhalation selon l'une des revendications 1 à 7, dans lequel la membrane (6) est réalisée en forme de disque et présente à son bord une collerette (34).

9. Dispositif d'inhalation selon l'une des revendications 5 à 8, dans lequel le piston de positionnement (12) comprend sur son côté frontal dirigé vers la membrane (6) plusieurs, de préférence quatre évidements (66, 68, 70, 72) ménagés en forme d'étoile en correspondance des barrettes (18, 20, 22, 24 ; 18', 20', 22', 24') du boîtier (4 ; 4'), l'un des évidements (72) étant plus long que les autres.

10. Dispositif d'inhalation selon l'une des revendications 1 à 9, dans lequel le piston de positionnement (12) est reçu avec étanchement dans le boîtier (4 ; 4').

11. Dispositif d'inhalation selon l'une des revendications 1 à 10, dans lequel la vis de positionnement (14 ; 14') est susceptible d'être fixée en direction axiale sur le piston de positionnement (12), et le piston de positionnement (12) est réglable en direction axiale par rapport au boîtier (4 ; 4') au moyen de la vis de positionnement (14 ; 14').

12. Dispositif d'inhalation selon l'une des revendications 5 à 11, dans lequel la vis de positionnement (14 ; 14') présente au moins une, de préférence deux ouverture(s) (74) dans la zone entre deux évidements voisins.

13. Dispositif d'inhalation selon la revendication 12, dans lequel il est prévu deux ouvertures respectives (74) entre deux évidements voisins (66, 68, 70, 72), les ouvertures (74) se trouvant sur une ligne périphérique commune et étant espacées l'une de l'autre d'environ 45°.

14. Dispositif d'inhalation selon l'une des revendications 5 à 13, dans lequel un canal d'écoulement central est prévu au moins par tronçons dans le boîtier (4 ; 4'), dans la membrane (6), dans la plaque de pression (8), dans l'élément obturateur (10) et dans le piston de positionnement (12).

15. Dispositif d'inhalation selon l'une des revendications 4 à 14, dans lequel la vis de positionnement (14) est vissée dans le boîtier (4).

16. Dispositif d'inhalation selon l'une des revendications 4 à 14, dans lequel la vis de positionnement (14') est reliée au boîtier (4') au moyen d'un guidage à coulisse (86, 90) pour guider axialement le piston de positionnement (12).

17. Valve réglable, en particulier pour l'utilisation dans un dispositif d'inhalation, comportant un boîtier (4 ; 4'), une membrane (6), un élément obturateur (10), le boîtier (4 ; 4') étant réalisé sensiblement tubulaire et comprenant plusieurs, de préférence quatre, barrettes (18, 20, 22, 24 ; 18', 20', 22', 24') agencées en étoile, **caractérisée en ce que** la valve comprend une plaque de pression optionnelle (8), un piston de positionnement (12) et une vis de positionnement (14 ; 14') pour régler le débit quantitatif à travers la valve réglable, et **en ce qu'**une barrette (24 ; 24') est plus longue que les autres, le boîtier (4 ; 4') étant réalisé, sur un côté des barrettes, pour le logement du piston de positionnement (12) avec possibilité de réglage et sur le côté opposé il est réalisé pour le logement de la membrane (6), de la plaque de pression optionnelle (8) et de l'élément obturateur (10).

18. Valve réglable selon la revendication 17, dans laquelle la membrane (6) est refoulée contre les barrettes (18, 20, 22, 24 ; 18', 20', 22', 24') du boîtier (4 ; 4') au moyen de la plaque de pression (8) de l'élément obturateur (10), de sorte qu'il se forme un espace volumétrique entre la membrane (6) et le piston de positionnement (12).

19. Valve réglable selon la revendication 17 ou 18, dans laquelle la membrane (6) est réalisée en forme de disque et présente à son bord une collerette (34).

20. Valve réglable selon l'une des revendications 17 à 19, dans laquelle le piston de positionnement (12) comprend sur son côté frontal dirigé vers la membrane (6) plusieurs, de préférence quatre, évidements (66, 68, 70, 72) ménagés en forme d'étoile en correspondance des barrettes (18, 20, 22, 24 ; 18', 20', 22', 24') du boîtier (4 ; 4'), l'un des évidements (72) étant plus long que les autres.

21. Valve réglable selon l'une des revendications 17 à 20, dans laquelle le piston de positionnement (12) est reçu avec étanchement dans le boîtier (4 ; 4).

22. Valve réglable selon l'une des revendications 17 à 21, dans laquelle la vis de positionnement (14 ; 14') est susceptible d'être fixée en direction axiale sur le piston de positionnement (12) et le piston de positionnement (12) est réglable en direction axiale par rapport au boîtier (4 ; 4') au moyen de la vis de positionnement (14 ; 14').

23. Valve réglable selon l'une des revendications 17 à 22, dans laquelle la vis de positionnement (14 ; 14') présente au moins une, de préférence deux, ouverture(s) (74) dans la zone entre deux évidements voisins.

24. Valve réglable selon la revendication 23, dans laquelle il est prévu deux ouvertures respectives (74) entre deux évidements voisins (66, 68, 70, 72), les ouvertures (74) se trouvant sur une ligne périphérique commune et étant espacées l'une de l'autre d'environ 45°.

25. Valve réglable selon l'une des revendications 17 à 24, dans laquelle un canal d'écoulement central est prévu au moins par tronçons dans le boîtier (4 ; 4'), dans la membrane (6), dans la plaque de pression (8), dans l'élément obturateur (10) et dans le piston de positionnement (12).

26. Valve réglable selon l'une des revendications 17 à 25, dans laquelle la vis de positionnement (14) est vissée dans le boîtier (4).

27. Valve réglable selon l'une des revendications 17 à 25, dans laquelle la vis de positionnement (14') est reliée au boîtier (4') au moyen d'un guidage à coulisse (86, 90) pour guider axialement le piston de positionnement (12).

28. Valve réglable selon l'une des revendications 17 à 27, qui est réalisée sous la forme d'un limiteur de flux variable en continu.

29. Valve réglable selon l'une des revendications 17 à 27, qui est réalisée sous la forme d'un limiteur de flux réglable à plusieurs étages.

30. Valve réglable selon l'une des revendications 17 à 29, dans laquelle la valve est raccordée à un dispositif de limitation volumétrique d'inhalation.

31. Valve réglable selon la revendication 30, dans laquelle la limitation volumétrique s'effectue directement ou indirectement en fonction du temps.
